# EUROPEAN PATENT APPLICATION

(11) **EP 0 709 071 A2**
(43) Date of publication of application: **01.05.1996**
(21) Application number: 95830434.7
(22) Date of filing: 13.10.1995
(51) Int. Cl.: A61F 2/36

(54) **Femoral prosthesis for reconditioning**

(30) Priority: 21.10.1994 IT BS940124
(71) Applicant: MEC HINT S.r.l., I-24060 Castelli Calepio (Bergamo) (IT)
(72) Inventor: Fiorini, Angela, I-17027 Pietra Ligure (Savona) (IT)
(74) Representative: Manzoni, Alessandro

(57) **Abstract**

A femoral prosthesis for re-implantation comprising a conical middle-distal stem (11) having high fins (14a) alternating with low fins (14b), wherein the spaces between the fins each have a concave bottom surface (15).

The prosthesis, moreover, has a proximal portion (12) with a wedge-shaped transverse cross-section and steps (16) affecting the external surface and the side surfaces of the portion.

## Description

The present invention pertains to femoral prostheses for reconditioning and re-implantation.

A careful examination of prosthetic hip surgery shows an increasing incidence of renewal, ie. of re-implantation, originating in various reasons that may be summarized as follows:
* Renewal of implants made when the prostheses available for the operators were very limiting, both as regards models and manufacturing technologies.
* Renewal of implants cemented with inadequate techniques.
* Renewal of implants with incorrect geometries, with obvious and frequent symptoms of pain.
* Renewal originating in the outcome of incorrect or failed osteotomy.

All this also in relation to the increased individual longevity highlighting the unchanged geometry of the prosthesis over time while there is a progressive variation in the bones with ageing.

Until some time ago, both industry and the reporting surgeons had mistakenly identified the problem of reconditioning with making implants that repeated the geometry of the prosthesis of the first implant, and only increasing the measurements of length and diameter, barely taking into account the profound structural and morphological modifications that had taken place in the receiving bone after removing the original prosthesis.

For these reasons, the prostheses for reconditioning most widely used were until recently nothing more than a larger version of the prosthesis of the first implant, with poor distal anchoring, or none at all, and therefore destined for early failure.

This complex problem, with poor historical literature, has been tackled by H. Wagner who designed a Femoral Prosthesis for Reconditioning, in a cone-shape, not cemented, (produced by Protek-Sulzer) for large losses of bone substance, which tends to solve some important aspects:
- the widening of the prosthesis seat;
- the narrowing of the receiving cortical bone, originating in reabsorption of the bone;
- bone deformity originating in previous osteotomies.

Wagner therefore focused his attention on the distal anchorage of the implant, therefore only in the area where there can be found a good bone-stock able to ensure a state of relative mechanical balance, trusting in sufficient ossification at the original prosthesis seat to compensate for the loss of bone matter.

A prosthesis for renewal called S-ROM (produced by Joint Medical Product Corp.) is also known with just a few others, and has a middle-distal stem longitudinally finned and a conical proximal portion, with a basically round cross-section and with circumferential steps extending along the entire perimeter of the proximal portion. It is an object of the present invention to more effectively and advantageously solve the problem of reconditioning or re-implanting a prosthetic system of the hip by means of a femoral prosthesis with new, original structural features so it can interact and better integrate with the bone in which it is implanted.

For this purpose, the femoral prosthesis according to the invention, which comprises a conical middle-distal stem with longitudinal fins, spaced angularly (equidistant) defining spaces between each other, and a proximal portion with steps widening towards the top from the said stem and supporting spherical head, is characterized in that the fins of the middle-distal stem comprise alternating high and low fins, the spaces between said fins each have a concave bottom surface, the proximal portion has a wedge-shaped transverse cross-section, defining an external surface, side surfaces and an internal surface and narrowing from the external surface towards the internal surface, the internal surface being set on the side of the spherical head, and the steps on said proximal portion affecting only the external surfaces and the sides of the portion itself.

Further details the invention will become apparent from the description of a preferred embodiment, made with reference to the accompanying, in wich:
Figure 1 shows by way of comparison,.a known femoral prosthesis for re-implantation;
Figure 1a is a transversal, partial and enlarged cross-sectional view of the middle-distal stem of the prosthesis of Fig. 1;
Figure 2 is a view of a single-block femoral prosthesis according to the invention;
Figure 2a is a transversal cross-sectional view of the middle-distal stem of the prosthesis of Fig. 2;
Figure 2b is a partial view of the end of the stem of the prosthesis of Fig. 2 to better highlight the shape of its fins;
Figure 2c is a side view of part of a fin from the stem of the prosthesis according to line II-II in Fig. 2a;

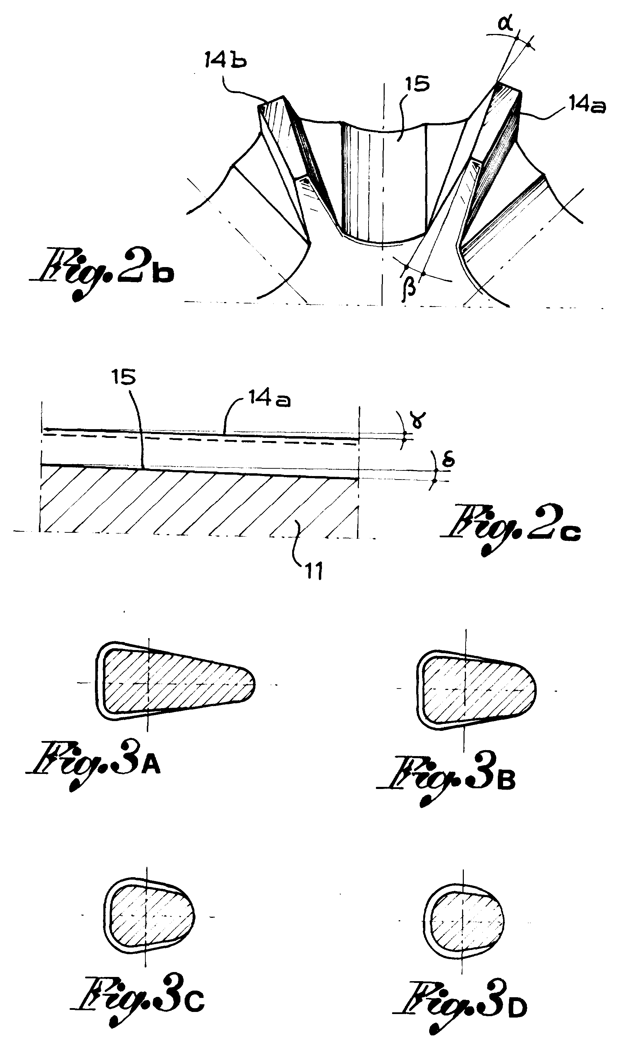

Figures 3A, 3B, 3C and 3D are respectively the transversal cross-sectional view according to the lines A, B, C and D, with respect to the level of some steps of the proximal portion;
Figure 4 shows the separate components of a two-portion prosthesis; and
Figure 5 shows the separate components of a three-portion prosthesis.

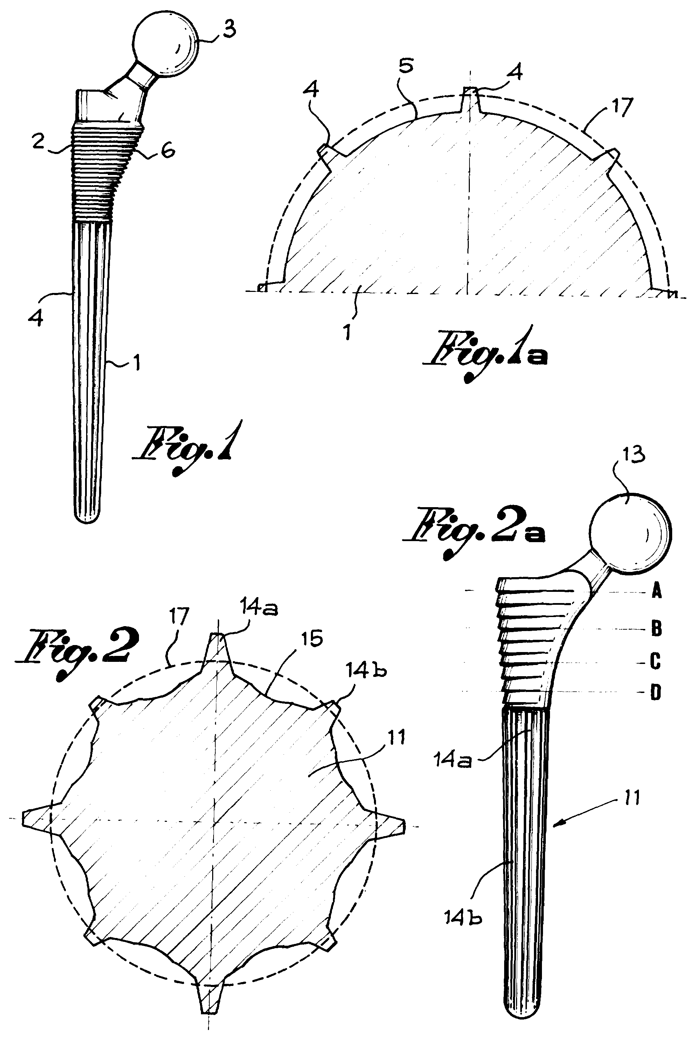

Figures 1 and 1a illustrate a known femoral prosthesis having a conical middle-distal stem 1 and a proximal portion 2 supporting, in the usual form, a spherical head 3 designed to replace the natural femoral head. The middle-distal stem of this prosthesis has equidistant longitudinal fins 4 all of the same height and together defining spaces with a convex bottom surface 5. The proximal portion 2 is basically round and carries a number of steps 6 that follow each other in height and which extend along the entire perimeter of the portion.

The femoral prosthesis of the present invention is illustrated in Figure 2; it has a conical middle-distal stem 11, finned longitudinally in a similar manner to the prosthesis described above for comparison, and a proximal portion 12 with a spherical head 13. However, this new prosthesis has some substantial differences with respect to the known prosthesis.

Its middle-distal stem 11 has, in its cross-section, higher longitudinal fins 14a that are alternated with lower fins 14b, and the spaces between the fins have a concave bottom surface 15. Eight equidistant fins may be provide, four high ones 14a and four low ones 14b, alternating as shown in Figure 2a.

The high fins 14a, denominated "intracortical" fins, have the main function of "biting" into the cortex of the femoral bone, while the low fins 14b, denominated "contact" fins, have a pre-eminently supporting function.

For these fins to be able to better perform the above mentioned functions, the supporting seat 17 of the stem 11 shall be arranged in the bone using a truncated/conical borer with a diameter of 0.5-1 mm less than the diameter of the contact fins 14b.

In relation to the patient's bone condition, the surgeon may make an adequate Press-Pit and in the course of the "life" of the re-implantation the fins 14a, 14b will definitely perform their functions which will be adapted to the regrowth of the bone, thus ensuring very high torsional stability in the distal portion as well.

In addition, said fins are conical, not only on the diameters as a consequence of the conical shape of the stem, but also on all their side faces, thereby achieving continuous axial support over time and relative to the load reaction.

Figures 1a and 2a show the difference between the homogeneous fins of the known prosthesis and the differentiated fins of the prosthesis according to the invention, as well as the diameter of the borer (dotted lines) that defines the supporting seat 17 of the stem of the prosthesis.

Figures 2b and 2c show the different side conicalness α and β and the longitudinal one γ on a section of length of the stem 11. Notice how the play of such conicalness positively fosters graft into the bone pin, meticulously avoiding undercutting and ensuring that the thickness of the bone graft is always much greater than the thickness of the fins of the prosthetic stem. Likewise, note the shape of the bottom surface 15 of the spaces, which besides being concave has a pronounced axial conicalness δ greater than the conicalness of the fins and of the seat borer: this is to facilitate wedging of the prosthesis, both in the action of the Press-Pit that the surgeon will do, and in the stabilization over time relative to the "life" of the prosthesis fostering bone regrowth.

The proximal portion 12 has a geometric shape that allows mechanical filling (prosthesis) in equilibrium with biological filling (bone chips). It has a wedge-shaped transverse cross-section with an internal surface, side surfaces and an external surface. The cross-section of the proximal portion narrows from the external surface towards the internal surface, the latter being on the side of the spherical head, ie. towards the axis of the person applying the prosthesis.

Said portion, moreover, has steps 16, in the form of saw teeth, with semi-sharp edges turned downwards to compress the filling bone chips and/or the natural bone itself. This structure, in the case of construction or regrowth of the Bone-Stock, hypothesises a definite possibility of renewal of the proximal anchorage

Notice that the steps 16 are provided on the external and side surfaces but are not provided on the "internal" portion, ie. on the side towards the axis of the person, where they would be dangerous and painful, besides being of no use.

In fact, it is precisely on this side that all the greater forces relative to the diaphysis angle are discharged and therefore supported.

It should be remembered that in a re-implantation, initial fixing is left to the distal and/or middle-distal portion, where there is normally a Bone-Stock, whereas the proximal portion is all or mostly to be rebuilt. Special bone conditions of the proximal portion could anyhow advise even combining the finned distal portion with a completely smooth proximal portion.

This invention takes these topics into account: with the special fins and the studied conicalness of the stem, it performs initial fixation and stabilization over time with the special geometry of the proximal portion.

Notice, likewise, the pronounced conicalness in the transverse cross-section of the proximal portion that reacts against the angular load applied onto the femoral head by the dead and/or walking weight of the patient, this conicalness also contributes, to quite an extent, to the stabilization at the start and over time of the element of synthesis.

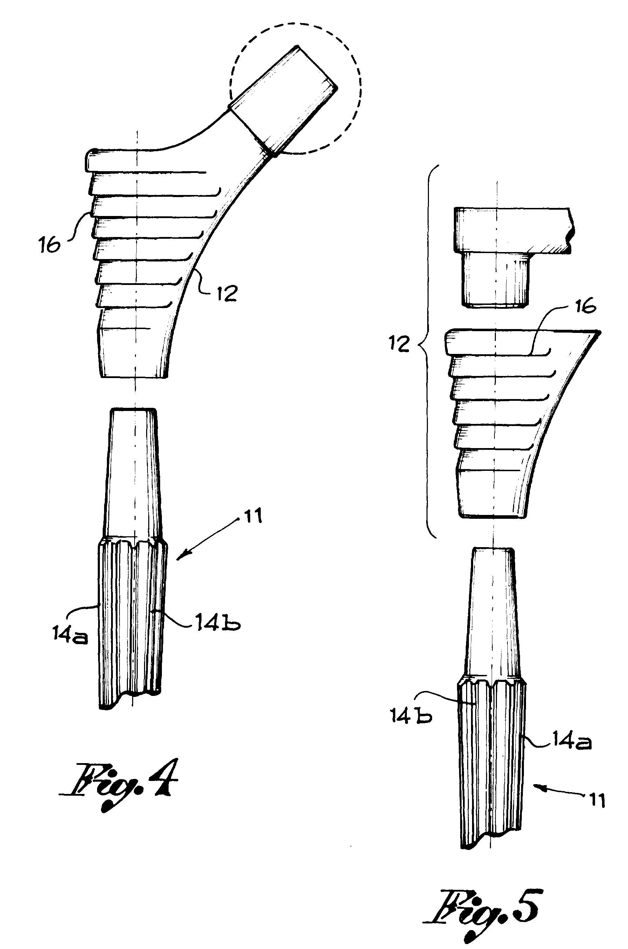

The invention may be accomplished in a single block, ie. in a single distal/proximal piece as shown in Fig. 2; or in two portions, distal plus proximal - see Fig. 4 - or even in three portions, distal/proximal plus femoral ball head - see Fig. 5. The assembly of the components of a two- or three-portion prosthesis may be made in a known manner or more advantageously with a system already described in a previous patent application made by the same applicant.

The surface finish may be smooth, with particular roughness, or with various and specific applications of films.

The various creations, with different dimensions or sizes combined with different angles and distances, permit giving satisfactory answer to all the requirements that may be found in a patient, both of dysplasia and of anteversion or of cervico-diaphysial angulation.

## Claims

1. A femoral prosthesis for re-implantation comprising a conical middle-distal stem (11) with longitudinal fins, spaced angularly defining spaces between them, and a stepped proximal portion (12) widening towards the top from said stem and supporting a spherical head (13), characterized in that the fins of the middle-distal stem comprise high fins (14a) alternating with low fins (14b), the spaces between said fins each have a concave bottom surface (15), the proximal portion (12) has a wedge-shaped transverse cross-section, defining an external surface, side surfaces, and an internal surface and narrowing from the external surface towards the internal surface, the internal surface being on the side of the spherical head (13), and the steps (16) on said proximal portion affecting only the external and side surfaces of the portion itself.

2. A femoral prosthesis according to claim 1, wherein every longitudinal fin (14a, 14b) of the middle-distal stem (11) has longitudinally and transversely conical side surfaces and wherein the concave bottom surface (15) of each space between said fins has a greater axial conicalness than that of the fins themselves.

3. A femoral prosthesis according to claim 1, wherein the steps of the proximal portion are stepped in saw-teeth and with semi-sharp edges turned downwards.

4. A femoral prosthesis according to claims 1-3, wherein the middle-distal stem and the proximal portion form a single body.

5. A femoral prosthesis according to claims 1-3, wherein the middle-distal stem and the proximal portion are two assembled components.

6. A femoral prosthesis according to claims 1-3, wherein the proximal portion is composed of two components that are fixed together and at the same time to the middle-distal stem.
